# EUROPÄISCHE PATENTSCHRIFT

(11) **EP 0 966 452 B1**
(45) Veröffentlichungstag und Bekanntmachung des Hinweises auf die Patenterteilung: **02.05.2003**
(21) Anmeldenummer: 98904041.5
(22) Anmeldetag: 08.01.1998
(51) Int. Cl.: C07D 261/04, C07D 261/20

(54) **VERFAHREN ZUR HERSTELLUNG VON SCHWEFELHALTIGEN 2-CHLOR-3-(4,5-DIHYDROISOXAZOL-3-YL)-BENZOESÄUREN**
PROCESS FOR PREPARING SULPHUROUS 2-CHLORO-3-(4,5-DIHYDROISOXAZOL-3-YL)-BENZOIC ACIDS
PROCEDE DE PREPARATION DE 2-CHLORO-3-(4,5-DIHYDROISOXAZOL-3-YLE)-ACIDES BENZOIQUES CONTENANT DU SOUFRE

(30) Priorität: 17.01.1997 DE 19701446; 06.03.1997 DE 19709118
(43) Veröffentlichungstag der Anmeldung: 29.12.1999
(73) Patentinhaber: BASF AKTIENGESELLSCHAFT, 67056 Ludwigshafen (DE)
(72) Erfinder: RHEINHEIMER, Joachim, D-67063 Ludwigshafen (DE); VON DEYN, Wolfgang, D-67435 Neustadt (DE); GEBHARDT, Joachim, D-67157 Wachenheim (DE); HILL, Regina, Luise, D-67346 Speyer (DE); RACK, Michael, D-69123 Heidelberg (DE); KÖNIG, Hartmann, D-69115 Heidelberg (DE); GÖTZ, Norbert, D-67547 Worms (DE); MAYWALD, Volker, D-67069 Ludwigshafen (DE); KARDORFF, Uwe, D-68159 Mannheim (DE)
(86) Internationale Anmeldenummer: EP9800066
(87) Internationale Veröffentlichungsnummer: WO98031676

(56) Entgegenhaltungen:
- WO-A-95/14680
- WO-A-96/26200
- WO-A-96/26206

## Beschreibung

Die Erfindung betrifft ein Verfahren zur Herstellung von schwefelhaltigen 2-Chlor-3-(4,5-dihydroisoxazol-3-yl)-benzoesäuren der allgemeinen Formel I 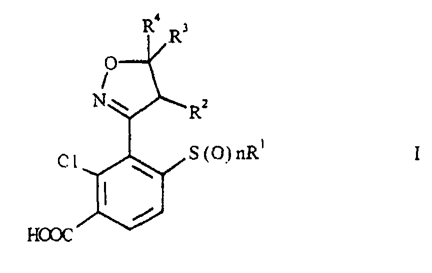 in der die Substituenten die folgende Bedeutung haben:
- n: 0, 1 oder 2;
- R¹: C₁-C₆-Alkyl, C₁-C₆-Halogenalkyl;
- R²,R³,R⁴: Wasserstoff, C₁-C₄-Alkyl, C₁-C₄-Halogenalkyl
oder
- R³ u. R⁴: bilden gemeinsam eine C₂-C₆-Alkandiyl-Kette, die ein- bis vierfach durch C₁-C₄-Alkyl substituiert sein kann.

Des weiteren betrifft die Erfindung die für dieses Verfahren wesentlichen Zwischenprodukte: Die Alkylthiobenzoesäuren der Formel Ia, Bromthioether der Formel V, Thioether der Formel IV und 3-(2,6-Dichlorphenyl)isoxazoline der Formel II.

Aus der WO 96/26200 ist bereits ein neunstufiges Verfahren zur Herstellung von 2-Chlor-3-(4,5-dihydroisoxazol-3-yl)-4-methylsulfonylbenzoesäuremethylester bekannt, bei dem der Aufbau der Isoxazolin-Einheit erst gegen Ende der Synthesesequenz erfolgt.

Des weiteren sind in der WO 96/26200 2,4-disubstituierte 3-(Heterocyclyl)benzoesäuren als Zwischenprodukte für Herbizide genannt. In der Wo 95/14680 und EP-A 245 825 sind 3-Phenylisoxazoline, die im Phenylkern zwei bis dreifach substituiert sein können, als Pharmazeutika beschrieben.

Aufgabe der vorliegenden Erfindung war es einen kürzeren, kostengünstigeren und großtechnisch anwendbaren Weg zu schwefelhaltigen 2-Chlor-3-(4,5-dihydroisoxazol-3-yl)-benzoesäuren der Formel I zu finden.

Demgemäß wurde das eingangs erwähnte Verfahren gefunden, das die gewünschten schwefelhaltigen 2-Chlor-3-(4,5-dihydroisoxazol-3-yl)-benzoesäuren der Formel I in guten Ausbeuten liefert (Schema 1). Alkylthiobenzoesäuren der Formel Ia sind somit in drei Stufen ausgehend von 3-(2,6-Dichlorphenyl)isoxazolinen der Formel II, bzw. in fünf Stufen ausgehend von dem käuflich erhältlichen 2,6-Dichlorbenzaldehyd zugänglich. Nach einem Oxidationsschritt erhält man dann die Alkylsulfonyl- oder Alkylsulfinyl-benzoesäuren der Formel Ib.

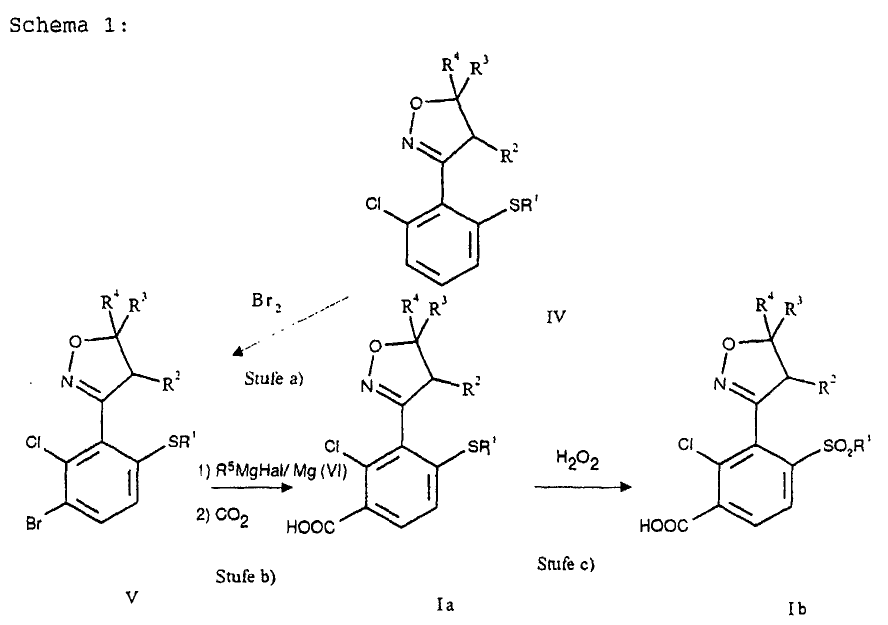

Das erfindungsgemäße Verfahren läßt sich zur Herstellung schwefelhaltiger 2-Chlor-3-(4,5-dihydroisoxazol-3-yl)-benzoesäuren der Formel I anwenden. Die vorstehend für die Substituenten R¹ bis R⁴ in der Formel I genannten Bedeutungen stellen Sammmelbegriffe für individuelle Aufzählungen der einzelnen Gruppenmitglieder dar. Sämtliche Kohlenstoffketten, also alle Alkylteile können geradkettig oder verzweigt sein. Halogenierte Substituenten tragen vorzugsweise 1 - 6 gleiche oder verschiedene Halogenatome.

### Im einzelnen bedeuten beispielsweise:

### C₁-C₄-Alkyl

Methyl, Ethyl, n-Propyl, 1-Methylethyl, n-Butyl, 1-Methylpropyl, 2-Methylpropyl und 1,1-Dimethylethyl;

### C₁-C₆-Alkyl

C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-2-methylpropyl;

### C₁-C₆-Halogenalkyl

C₁-C₆-Alkyl wie vorstehend genannt, das partiell oder vollständig durch Fluor, Chlor und/oder Brom substituiert ist, also z. B., Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 3-Chlorpropyl, vorzugsweise Trifluormethyl.

Besonders bevorzugt sind Alkylthiobenzoesäuren der Formel I, Bromthioether der Formel V, Thioether der Formel IV und 3-(2,6-Dichlorphenyl)isoxazoline der Formel II, wobei die Subscituenten R² bis R⁴ jeweils Wasserstoff bedeuten.

Das Verfahren ist aufgrund der verwendeten Reagenzien und der Reaktionsbedingungen auch großtechnisch anwendbar (scale up-fähig).

Darüberhinaus wurden schwefelhaltige 2-Chlor-3-(4,5-dihydroisoxazol-3-yl)-benzoesäuren der Formel Ia, Bromthioether der Formel V, Thioether der Formel IV und die 3-(2,6-Dichlorphenyl)isoxazoline der Formel II gefunden, die sich als Zwischenprodukte eignen.

Die im erfindungsgemäßen Verfahren benötigten Thioether der Formel IV können auf verschiedene Weise hergestellt werden (Schema 2). Der Weg C ist hierbei besonders bevorzugt, da die Thiolyse zu den Thioethern IV bei höherer Temperatur (0 bis 120°C) als die entsprechenden Thiolysereaktionen der Verfahren A und B ablaufen kann, ohne daß größere Mengen des unerwünschten doppelt thiolierten Nebenproduktes gebildet werden.

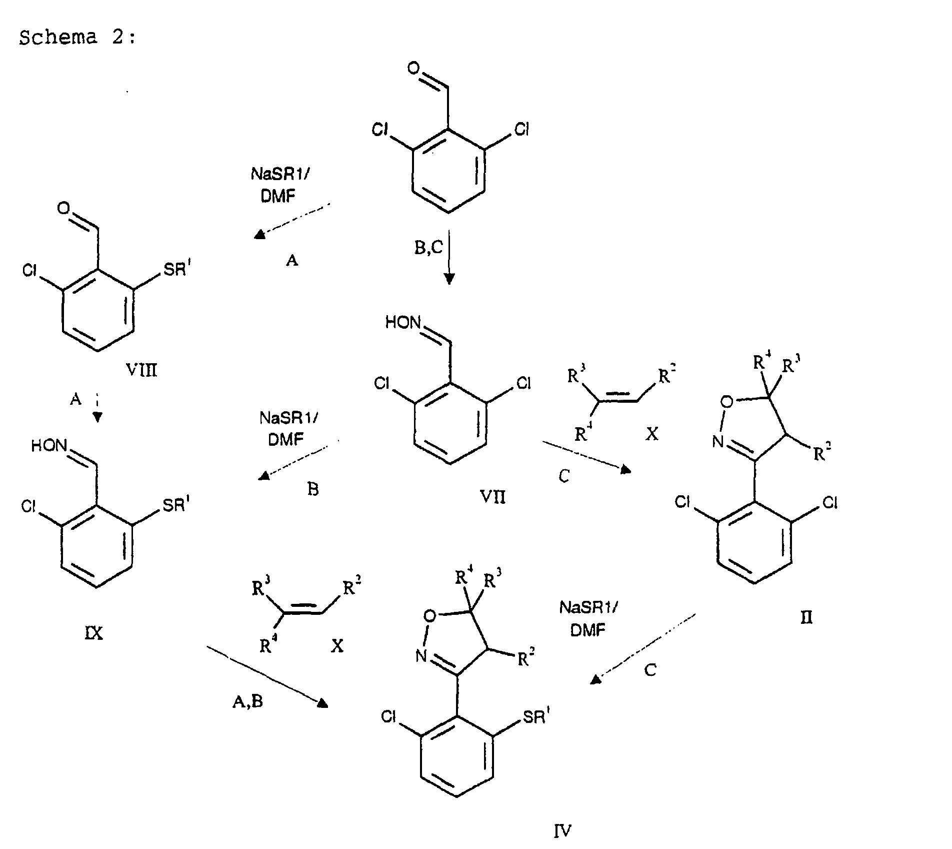

Die 2,6-Dichlorbenzaldoxim der Formel VII ist nach Standardverfahren ausgehend von käuflich erwerblichen 2,6-Dichlorbenzaldehyd VIIa durch Umsetzung mit Hydroxylamin in Gegenwart von Säure in nahezu quantitativer Ausbeute zugänglich.

Die Umsetzung des 2,6-Dichlorbenzaldoxims VII mit Alkenen der Formel X zu II verläuft über verschiedene Zwischenstufen. Da der erste Reaktionsschritt die Bildung eines intermediären Hydroximsäurehalogenides ist, müssen ein geeignetes oxidierendes Reagenz und eine Halogenquelle oder auch das Halogen selbst zugegen sein. Der zweite Reaktionsschritt ist dann die Eliminierung von Halogenwasserstoff zum Nitriloxid, die basische Bedingungen erfordert. Als Drittes folgt schließlich die Cycloaddition des Nitriloxids an das Alken.

Nach üblichen Verfahren läßt sich diese Sequenz schrittweise ausführen, wobei zur Bildung des Hydroximsäurehalogenids z. B. die freien Halogene Brom oder Chlor eingesetzt werden können. Da die Hydroximsäurehalogenide zur Zersetzung neigen, werden sie vorteilhaft mit einer Base weiter in die noch empfindlicheren Nitriloxide übergeführt, die man meist in situ mit dem Alken abfängt.

Bei dem erfindungsgemäßen Verfahren wurden diese Einzelschritte nun vorteilhaft zu einer "Eintopfreaktion" zusammengefaßt. Dazu wird in der Regel in einem Lösungsmittel wie beispielsweise Halogenalkanen wie Dichlorethan und Methylenchlorid oder Aromaten wie Benzol, Toluol, Chlorbenzol, Nitrobenzol oder Xylol gearbeitet, daß die organische Komponente löst, aber selbst nicht störend in die Reaktion eingreift. Als Halogenierungsmittel und gleichzeitig als Base wird eine wässrige Alkalihypohalogenit-Lösung, vorzugsweise 1-2 Äquivalente kommerziell verfügbare Natriumhypochlorit-Lösung, zugegeben, wobei das Alken parallel oder unmittelbar danach zugefügt wird. Das Reaktionsgemisch ist also üblicherweise zweiphasig, da sich das organische Lösungsmittel mit der Alkalihypohalogenit-Lösung nur unvollständig mischt. Zur Vervollständigung des Umsatzes kann es vorteilhaft sein, 3-50% Natrium- oder Kaliumacetat zuzugeben, dies ist jedoch nicht unbedingt erforderlich.

Gasförmige Alkene der Formel X werden eingeleitet, flüssige Alkene entsprechend zudosiert. Die Alkene werden in der Regel in einem Molverhältnis von 1 bis 5 : 1 in Bezug auf das Oxim VII eingesetzt.

Die Reaktion wird im allgemeinen bei Temperaturen von 0 bis 80°C, bevorzugt bei Temperaturen zwischen 20 und 50°C durchgeführt.

Die Thiolyse der so erhaltenen 3-(2,6-Dichlorphenyl)isoxazoline II wird mit einer Thiolverbindung der Formel III

R¹SH III,

bevorzugt jedoch mit deren Alkalisalz IIIa durchgeführt.

Anstelle von IIIa kann in der Regel auch die Thiolverbindung III unter Zusatz von Basen wie Alkali- oder Erdalkalicarbonaten, Alkali- oder Erdalkalihydroxiden oder Alkalialkoholaten eingesetzt werden. Durch den Zusatz von Kupferpulver als Katalysator (0,01-10 mol%) läßt sich oftmals eine Vervollständigung und Beschleunigung der Umsetzung erreichen.

Polare Lösungsmittel haben sich für diese Reaktion als geeignet erwiesen. Besonders bevorzugt sind polare, aprotische Lösungsmittel wie Dimethylformamid, Dimethylacetamid, N-Methylpyrrolidon, Dimethylpropylenharnstoff, Tetramethylharnstoff.

Die Thiolyse wird in der Regel bei 0 bis 100°C, bevorzugt bei 20 -50°C durchgeführt.

Die einzelnen Stufen der Wege A und B können analog den jeweiligen des Weges C ausgeführt werden. Zu beachten ist lediglich, daß die Thiolyse von 2,6-Dichlorbenzaldehyd zu VIII (Weg A) in der Regel zwischen -30 und 30°C, vorzugsweise zwischen -20 und 0°C und die Thiolyse von VIIb zu IX (Weg B) bei Temperaturen von -10 bis 80°C , vorzugsweise bei 0 bis 60°C erfolgen sollte.

Für die Bromierung der Thioether IV zu den Bromthioechern V eignen sich Bromierungsmittel wie elementares Brom, NBS und Dibromdimethylhydantoin.

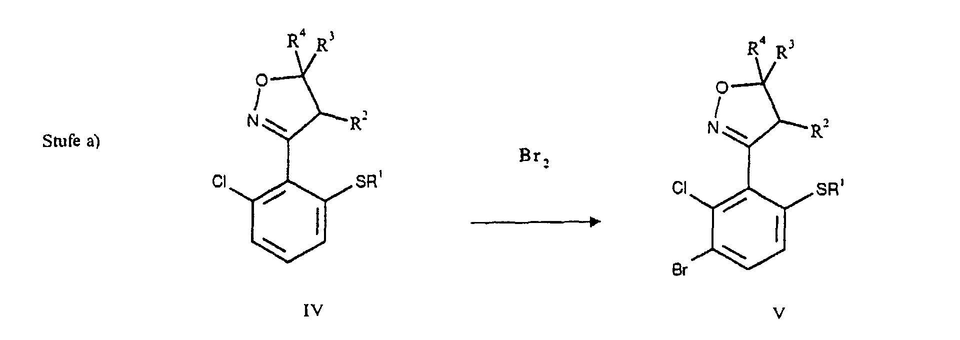

Als besonders geeignet hat sich elementares Brom in Gegenwart von konzentrierter Schwefelsäure erwiesen. Dabei können sogar beide Bromatome in das Produkt eingebracht werden. Üblicherweise kommen 0,5 bis 0,7 Äquivalente, vorzugsweise 0,5 bis 0,6 Äquivalente Brom zum Einsatz.

Bromierungen in konzentrierter Schwefelsäure waren bisher nur an desaktivierten, aber säure- und hydrolysestabilen Derivaten wie Dinitrobenzol beschrieben worden (Monatsh. für Chem. 99 (1968), 815 - 22). Die vorliegenden Thioether VI weisen jedoch mit der Thioether- und Isoxazolin-Gruppierung gleich zwei oxidations- bzw. hydrolyseempfindliche Gruppierungen auf. Dennoch verläuft die Reaktion überraschenderweise chemoselektiv. So bilden sich bei geeigneter Reaktionsführung weniger als 5% Nebenprodukte wie beispielsweise das entsprechende Sulfoxid oder Hydrolyseprodukte.

Die Reaktion wird in der Regel bei Temperaturen von -10 bis 80°C, vorzugsweise bei 0 bis 50°C ausgeführt.

Als Katalysator kann beispielsweise Schwefel, Iod oder Dibromdimethylhydantoin zugesetzt werden, in der Regel ist dies jedoch nicht erforderlich.

Alternativ zur Schwefelsäure können auch andere Lösungsmittel wie beispielsweise C₁-C₄-Carbonsäuren und hier insbesondere Essigsäure zum Einsatz gelangen.

Bei der hydrolytischen Aufarbeitung der in konzentrierter Schwefelsäure durchgeführten Bromierung werden erhebliche Wärmemengen frei. Dennoch sollte der Reaktionsansatz bei der Aufarbeitung unter 80°C, vorzugsweise unter 50°C gehalten werden, um mögliche Nebenreaktionen weitgehend zu verhindern.

Die Carboxylierung der Bromthioether V zu den Alkylthiobenzoesäuren der Formel Ia verläuft über die entsprechenden, intermediär gebildeten Arylgrignardverbindungen. Die Arylgrignardverbindungen werden durch Umsetzung der Bromthioether V mit Magnesium bzw. mit einer Alkylgrignardverbindung der Formel VI, wobei R⁵ die Bedeutung C₁-C₆-Alkyl und Hal die Bedeutung Chlor, Brom oder Iod haben gebildet. Besonders bevorzugt ist hierbei Isopropylmagnesiumchlorid.

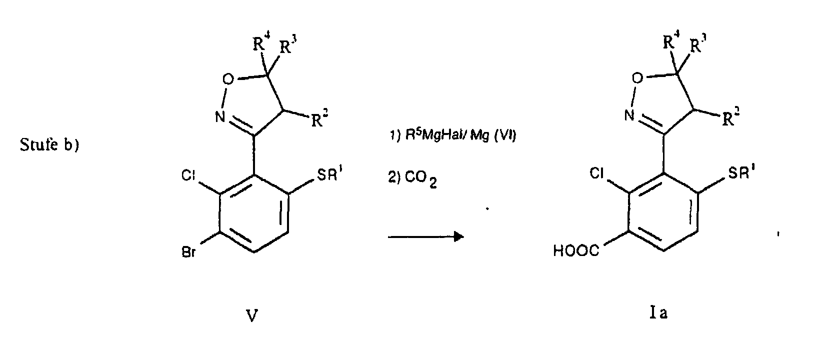

Die Bildung der Arylgrignardverbindung erfolgt selektiv an der Stelle des Bromatoms. Nebenreaktionen, die auf die intermediäre Bildung eines Arins - aufgrund des Chloratoms in Nachbarstellung - hindeuten würden, wurden nicht beobachtet. Alkylsulfonylreste hingegen sind unter den gewählten Reaktionsbedingungen gegenüber Grignardreagentien nicht inert. Als ein weiterer, vorteilhafter Aspekt des erfindungsgemäßen Verfahrens erweist sich daher, daß zur Herstellung der Sulfoxide und Sulfone der Formel Ib die Alkylsulfonyleinheit erst im Anschluß an die Carbonsäurefunktion aufgebaut wird.

Zur Herstellung der Arylmagnesiumverbindung kann auf die Alkylmagesiumverbindung der Formel VI teilweise oder ganz verzichtet und anstelle dessen Magnesiumpulver eingesetzt werden.

Bei ausschließlicher Verwendung von Magnesiumpulver kann dieses zunächst mittels einer der literaturbekannten Methoden aktiviert werden (Organikum, 1993 Barth Verlagsgesellschaft Leipzig, S. 518), wobei Isopropylchlorid oder Isopropylbromid besonders gut geeignet sind. Eine weitere bevorzugte Möglichkeit, das Magnesiumpulver zu aktivieren ist es, dem Pulver 0,1 bis 30, vorzugsweise 3 bis 15 mol% einer Isopropylmagnesiumchlorid-Lösung zuzusetzen.

Magnesiumpulver wird im allgemeinen in einem Molverhältnis von 0,9 bis 2 zum Bromthioether V eingesetzt; die Alkylmagnesiumverbindungen der Formel VI in einem Molverhältnis von 0,9 bis 3, vorzugsweise 1,0 bis 2,0.

Die Reaktion wird in unter den Reaktionsbedingungen inerten Lösungsmitteln durchgeführt. Besonders bevorzugt sind Ether wie Tetrahydrofuran, Diethylether, Dimethoxyethan oder Methyl-tert.butylether. In der Regel wird eine Reaktiontemperatur von -10 bis 80°C und bevorzugt von 10 bis 60°C eingestellt.

Die Carboxylierungsreaktion wird in der Regel durch Zusatz von gasförmigem oder festem Kohlendioxid im Molverhältnis von 1 bis 10 in Bezug auf den eingesetzten Bromthioether V durchgeführt.

Die Oxidation der Alkylthiobenzoesäuren Ia zu den entsprechenden Alkylsulfonyl- oder Alkylsulfinylbenzoesäuren der Formel Ib wird bevorzugt mit Wasserstoffperoxid durchgeführt, wobei mit etwa äquivalenten Mengen an Oxidans die Sulfoxide und mit etwa doppelt molaren Mengen die Sulfone erhalten werden.

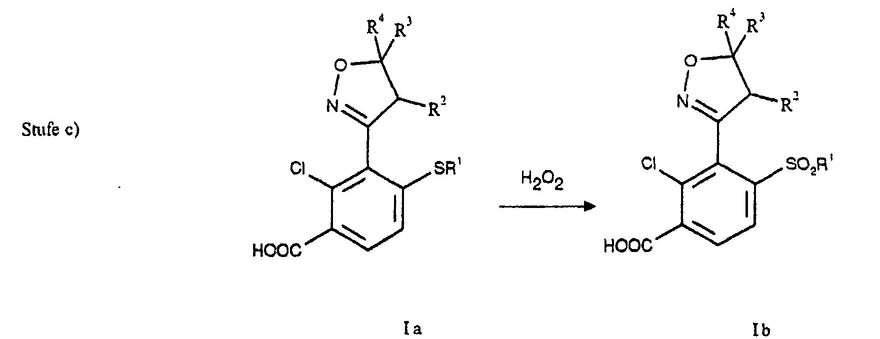

Als Lösungsmittel können Wasser, Acetonitril, Carbonsäuren wie Essigsäure, Trifluoressigsäure, Propionsäure, Alkohole wie Methanol, Ethanol, Isopropanol, tert.-Butanol, chlorierte Kohlenwasserstoffe wie Methylenchlorid, 1,1,2,2-Tetrachlorethan oder Ketone wie Aceton oder Methylethylketon verwendet werden. Besonders bevorzugt sind Wasser, Methanol, Essigsäure und Trifluoressigsäure.

In einer besonders bevorzugten Variante kann die Reaktion auch durch Zugabe stärkerer Säuren wie Trifluoressigsäure oder Perchlorsäure katalysiert werden. Als Katalysatoren sind jedoch auch Metallverbindungen geeignet, z. B. Übergangsmetalloxide wie Vanadinpentaoxid, Natriumwolframat, Kaliumdichromat, Eisenoxidwolframat, Natriumwolframat-Molybdänsäure, Osmiumsäure, Titantrichlorid, Selendioxid, Phenylenselensäure, Oxovanadinyl-2,4-pentandionat.

Die Katalysatoren werden im allgemeinen in einer Menge von 0,5 bis 10 % eingesetzt, wegen der leichten Filtrierbarkeit und Wiedergewinnung der anorganischen Katalysatoren können jedoch auch stöchiometrische Mengen eingesetzt werden.

Ein weiteres bevorzugtes Oxidationsmittel ist Peressigsäure oder Wasserstoffperoxid / Acetanhydrid, gegebenenfalls auch die in einer Wasserstoffperoxid / Essigsäure-Mischung im Gleichgewicht vorhandene peressigsäure.

Ein bevorzugtes Oxidationsmittel stellt auch die Pertrifluoressigsäure bzw. die Mischung Wasserstoffperoxid / Trifluoressigsäure oder auch die Mischung Wasserstoffperoxid / Trifluoracetanhydrid dar.

Die Oxidation mit Wasserstoffperoxid in Eisessig ist im allgemeinen sehr selektiv, jedoch häufig langsam. Durch Zugabe von Trifluoressigsäure kann die Reaktionszeit im allgemeinen verkürzt werden.

Als Lösungsmittel können weiterhin Petrolether, die vorgenannten Lösungsmittel sowie die oben aufgeführten Katalysatoren verwendet werden.

Neben Peressigsäure und Pertrifluoressigsäure können auch, Perbenzoesäure, Monoperphthalsäure oder 3-Chlor-perbenzoesäure zweckmäßig in chlorierten Kohlenwasserstoffen wie Methylenchlorid oder 1,2-Dichlorethan eingesetzt werden.

Sehr geeignet zur Oxidation der Thiole zu Sulfoxiden oder Sulfonen sind ferner Chlor und Brom. Günstig sind als Lösungsmittel Wasser, Acetonitril, Dioxan, Zweiphasensysteme wie wäßrige Kaliumhydrogencarbonatlösung / Dichlormethan sowie Essigsäure.

Als Quelle für aktives Halogen können ferner tert.-Butylhypochlorit, Unterchlorige sowie Unterbromige Säure, deren Salze, ferner N-Halogenverbindungen wie N-Brom- und N-Chlorsuccinimid oder auch Sulfurylchlorid eingesetzt werden.

Günstig für die Oxidation sind ferner Distickstofftetroxid z. B. in der verfahrenstechnisch einfachen Variante mit Luft/Stickstoffdioxid bzw. -trioxid und beispielsweise Osmium(VIII)-oxid als Katalysator. Daneben kann die Oxidation auch direkt mit Salpetersäure durchgeführt werden, wobei als zusätzliche Lösungsmittel Acetanhydrid, Essigsäure und als Katalysatoren Kupfer(I) und (II)-bromid und -chlorid in Frage kommen.

Geeignet für die Oxidation ist auch die photosensibilisierte Sauerstoffübertragung, wobei als Photosensibilisatoren Chlorophyll, Protoporphyrin, Rose Bengale oder Methylenblau zu empfehlen sind. Als inerte Lösungsmittel sind Kohlenwasserstoffe wie Pentan, Hexan, Heptan, Cyclohexan, chlorierte Kohlenwasserstoffe wie Methylenchlorid, 1,2-Dichlorethan, 1,1,2,2-Tetrachlorethan, Alkohole wie Methanol, Ethanol, n-Propanol oder Isopropanol, Ketone wie Aceton, Methylethylketon, polare aprotische Lösungsmittel wie Acetonitril, Propionitril oder aromatische Kohlenwasserstoffe wie Benzol, Toluol, Chlorbenzol oder Xylol geeignet. An Stelle von Sauerstoff kann man auch Ozon verwenden in den obengenannten Lösungsmitteln, zusätzlich noch Ether, 1,4-Dioxan oder THF.

Neben der Photosensibilisierung empfehlen sich für die Sauerstoffoxidation auch Katalysatoren z. B. Oxide und Sulfide von Nickel, Kupfer, Aluminium, Wolfram, Chrom, Vanadium, Ruthenium, Titan, Mangan, Molybdän, Magnesium und Eisen.

Je nach Stöchiometrie der verwendeten Oxidationsmittel gelangt man entweder zu den Sulfoxiden oder Sulfonen Ib. Die molaren Verhältnisse, in denen die Ausgangsverbindungen miteinander umgesetzt werden, betragen im allgemeinen 0,9 - 1,8, vorzugsweise 1,05 - 1,3 für das Verhältnis von Alkylthiobenzoesäure Ia zu Oxidationsmittel im Falle der Oxidation zur Alkylsulfinylbenzoesäure Ib (m=0) und im allgemeinen 1,9 - 3,5, vorzugsweise 2,05 - 2,9 im Falle der Oxidation zur Alkylsulfinylbenzoesäure Ib (m=1).

Die Konzentration der Edukte im Lösungsmittel beträgt im allgemeinen 0,1 - 5 mol/l, bevorzugt 0,2 - 2 mol/l.

Vorteilhaft legt man die Alkylthiobenzoesäure mit einem der vorgenannten Katalysatoren in einem der vorgenannten Lösungsmittel vor und gibt dann das Oxidationsmittel während 0,25 - 20 Stunden unter Rühren hinzu.

Die Oxidationen können drucklos oder unter Druck, kontinuierlich oder diskontinuierlich betrieben werden.

Die erfindungsgemäßen Alkylthiobenzoesäuren Ia und auch die damit zugänglichen Alkylsulfonylbenzoesäuren Ib sind wertvolle Vorprodukte für die Herstellung von Pflanzenschutzmitteln, insbesondere Herbiziden, wie sie in WO 96/26200, WO 96/26192, WO 96/26193 und WO 96/26206 beschrieben werden.

Im Folgenden wird das erfindungsgemäße Verfahren an Hand von Beispielen vorgestellt.

### Herstellung der Ausgangsmaterialien:

### Beispiel 1

### Herstellung von 2-Chlor-6-methylthiobenzaldehyd

20,0 g (0,114 Mol) 2,6-Dichlorbenzaldehyd wurden in 100 ml Dimethylformamid gelöst und unter Stickstoff-Schutzgas bei -15°C unter Rühren mit 7,54 g (0,107 Mol) Natriummethylthiolat versetzt. Es wurde 5 h bei -10 bis -15°C und dann 12 h bei Raumtemperatur nachgerührt. Die Reaktionsmischung wurde in Methyltert.-butylether aufgenommen, mit Wasser gewaschen, über Natriumsulfat getrocknet und im Vakuum eingeengt. Ausbeute 19,6 g (92%) Feststoff.
Reinheit: >95% (NMR, GC). ¹H-NMR (CDCl₃, ppm): δ = 2,47 (s); 7,23 (m); 7,44 (t); 10,66 (s) .

Bei einer durch Chromatographie an Kieselgel gereinigten Probe wurde ein Schmelzpunkt von 82°C gemessen.

### Beispiel 2

### Herstellung von 2-Chlor-6-methylthiobenzaldoxim

69,2 g (0,371 Mol) 2-Chlor-6-methylthiobenzaldehyd wurden in 500 ml Methanol gelöst und bei Raumtemperatur mit 93,5 g (1,11 Mol) Natriumhydrogencarbonat und 91,3 g (0,557 Mol) Hydroxylammoniumsulfat versetzt. Es wurde 12 h bei Raumtemperatur nachgerührt, vom unlöslichen Anteil abfiltriert und im Vakuum eingeengt. Der Rückstand wurde in Methyl-tert.-butylether aufgenommen, mit Wasser gewaschen, über Natriumsulfat getrocknet und im Vakuum eingeengt. Ausbeute 69,0 g (92,3 %) Feststoff vom Schmp. 143°C. Reinheit: >95% (NMR). ¹H-NMR (CDCl₃, ppm): δ = 2,47 (s); 7,10-7,30 (m); 8,58 (s); 8,67 (s).

### Beispiel 3

### Herstellung von 2,6-Dichlorbenzaldoxim

50,0 g (0,286 Mol) 2,6-Dichlorbenzaldehyd wurden in 250 ml Tetrahydrofuran und 250 ml Wasser gegeben und mit 26,0 g (0,159 Mol) Hydroxylammoniumsulfat versetzt. Dann wurde 3 h bei 40°C gerührt, wobei der pH-Wert durch Zugabe von Natriumhydrogencarbonat-Lösung bei 4-5 gehalten wurde. Anschließend wurde das Tetrahydrofuran im Vakuum abdestilliert, mit Methyl-tert.-butylether extrahiert, über Natriumsulfat getrocknet und im Vakuum eingeengt. Ausbeute 54,3 g (99,9 %) Feststoff vom Schmp. 150°C.
Reinheit: >97% (NMR). ¹H-NMR (CDCl₃, ppm): δ = 7,20 - 7,40 (m); 8,42 (s); 9,10 (br.).

### Herstellung der 3-(2,6-Dichlorphenyl)isoxazoline der Formel II

### Beispiel 4

### Herstellung von 3-(2,6-Dichlorphenyl)-4,5-dihydroisoxazol

a) 10,0 g (52,6 mmol) 2,6-Dichlorbenzaldoxim wurden in 150 ml Dichlormethan suspendiert. Nach dem Beginn der Ethylen-Einleitung wurden bei 15-20°C 32,7 ml (64,3 mmol) 12%ige Natriumhypochlorit-Lösung versetzt mit einer Spatelspitze Natriumacetat zugetropft. Nach dem Ende der Zugabe wurde auf 26-30°C erwärmt und noch 6 h Ethylen eingeleitet. Anschließend wurde die organische Phase abgetrennt, mit Wasser gewaschen, über Natriumsulfat getrocknet und im Vakuum eingeengt. Auswaage 10,5 g (92,4%) Feststoff vom Schmp. 100-101°C. Reinheit 99,0% (GC). ¹H-NMR (CDCl₃): δ = 3,31 (t); 4,56 (t); 7,25-7,45 (m).
   Ein analoger Versuch mit 20,8 g 2,6-Dichlorbenzaldoxim erbrachte eine Ausbeute von 93% bei einer Reinheit von 99,2% (GC).
b) 31,4 g (0,165 Mol) 2,6-Dichlorbenzaldoxim wurden in 350 ml 1,2-Dichlorethan suspendiert. Bei Raumtemperatur wurden 103 ml (0,202 Mol) 12%ige Natriumhypochlorit-Lösung zugegeben, wobei sich die Mischung auf 45°C erwärmte. Anschließend wurde bei Rückflußtemperatur 2 h Ethylen eingeleitet, eine Spatelspitze Natriumacetat zugegeben und nochmals 2 h Ethylen eingeleitet. Die organische Phase wurde abgetrennt, über Natriumsulfat getrocknet und im Vakuum eingeengt. Auswaage 31,6 g (88,7%) Feststoff.
   Reinheit 98,2% (GC).
c) Ausgehend von 2,6-Dichlorbenzaldehyd 75g (0.43mol) 2,6-Dichlorbenzaldehyd wurden in 400ml Toluol gelöst und anschließend mit 155g (0,24mol) 25%iger Hydroxylammoniumsulfatlösung versetzt. Bei 75°C wurden 38g 50%ige NaOH zugetropft. Nachdem eine halbe Stunde nachgerührt worden war, wurde bei 75°C eine Phasentrennung durchgeführt und die organische Phase mit Wasser gewaschen. Die organische Phase wurde abgekühlt. Über 4-6 h wurden bei 25°C 276g (0,46mol) 12,5%ige Natriumhypochloritlösung zugetropft und gleichzeitig Ethylen eingeleitet. Nach vollständiger Zugabe der Hypochloritlösung wurde 1 h nachgerührt, die Phasen getrennt, die organische Phase mit Wasser gewaschen und das Lösungsmittel im Vakuum abdestilliert. Man erhielt 82g (89% d. Th.) des Wertproduktes mit einer Reinheit (lt. GC) von 96%.

### Herstellung der Thioether der Formel IV

### Beispiel 5

### Herstellung von 3-(2-Chlor-6-methylthiophenyl)-4,5-dihydroisoxazol

a) Ausgehend von 2-Chlor-6-methylthiobenzaldoxim 57,5 g (0,285 Mol) 2-Chlor-6-methylthiobenzaldoxim wurden in 600 ml Dichlormethan suspendiert. Nach dem Beginn der Ethylen-Einleitung wurden bei 20-25°C 180 ml (0,354 Mol) 12%ige Natriumhypochlorit-Lösung zugetropft. Nach dem Ende der Zugabe wurde auf 33°C erwärmt und noch 5 h Ethylen eingeleitet. Danach wurden 2 Spatelspitzen Natriumacetat hinzugefügt und nochmals 30 min Ethylen eingeleitet. Anschließend wurde die organische Phase abgetrennt, mit Wasser gewaschen, über Natriumsulfat getrocknet und im Vakuum eingeengt. Auswaage 59,2 g (91%) gelblicher Feststoff vom Schmp. 62-64°C.
   Reinheit 90% (NMR). Ausbeute (Auswaage x Reinheit) ca. 81%. ¹H-NMR (CDCl₃): δ = 2,45 (s); 3,33 (t); 4,55 (t); 7,15-7,35 (m).
b) Ausgehend von 3-(2,6-Dichlorphenyl)-4,5-dihydroisoxazol 2,0 g (9,26 mmol) 3-(2,6-Dichlorphenyl)-4,5-dihydroisoxazol gelöst in 20 ml Dimethylformamid wurden bei Raumtemperatur mit 0,68 g (9,72 mmol) Natriummethylthiolat versetzt und 3 h bei 35°C gerührt. Anschließend wurde die Mischung mit Methyltert.-butylether verdünnt, mit Wasser gewaschen, über Natriumsulfat getrocknet und im Vakuum eingeengt. Auswaage 2,1 g (99,7%). Nach GC waren 88,7% Produkt und 9,4% Ausgangsmaterial enthalten.
bb) 200g (0,93mol) 3-(2,6-Dichlorphenyl)-4,5-dihydroisoxazol wurden in 620ml N-Methylpyrrolidon gelöst. Die Apparatur wurde evakuiert (100-20mbar) und anschließend wurde bei 60-65°C 330g (1,02mol) 21%ige methanolische Thiomethylatlösung zugefahren und das Methanol dabei abdestilliert. Nach Ende der Methanoldestillation wurde 1h nachgerührt und das Reakcionsgemisch anschließend mit 1,81 Wasser verdünnt und mit Toluol extrahiert. Die organische Phase wurde mit Wasser gewaschen und anschließend wurde das Toluol im Vakuum abdestilliert. Nach Umkristallisation aus Isopropanol erhielt man 169g (80% d. Th.) Wercprodukt mit einer Reinheit (nach GC) von 95%.

### Beispiel 6

### Herstellung von 2-Chlor-3-(4,5-dihydroisoxazol-3-yl)-4-methylsulfonylbenzoesäure

### Stufe a:

### Herstellung von 3-(3-Brom-2-chlor-6-methylthiophenyl)-4,5-dihydroisoxazol

a) Zu 4,0 g (17,6 mmol) 3-(2-Chlor-6-methylthiophenyl)-4,5-dihydroisoxazol gelöst in 30 ml conc. Schwefelsäure wurden bei Raumtemperatur 1,54 g (9,6 mmol) Brom langsam zugetropft, wobei sich die Mischung leicht erwärmte. Es wurde 1 h nachgerührt, auf 0°C abgekühlt und innerhalb von 30 min vorsichtig mit 200 ml Eiswasser versetzt, wobei die Temperatur unter 25°C gehalten wurde. Nach 15-minütigem Nachrühren wurde mit Essigsäureechylester extrahiert, die organische Phase mit wasser gewaschen, über Natriumsulfat getrocknet und im Vakuum eingeengt. Ausbeute 4,45 g (82,6%) Feststoff. Reinheit >95% (GC). ¹H-NMR (CDCl₃): δ = 2,45 (s); 3,30 (t); 4,58 (t); 7,05 (d); 7,63 (d).
aa) 45,0 g (0,198 Mol) 3-(2-Chlor-6-methylthiophenyl)-4,5-dihydroisoxazol wurden bei 5°C in 310 ml conc. Schwefelsäure gelöst. Dann wurden 17,42 g (109 mmol) Brom innerhalb von 3 min zugegeben, wobei sich die Mischung auf 8°C erwärmte. Es wurde 1,5 h bei 0-5°C und 45 min bei Raumtemperatur nachgerührt, auf 0°C abgekühlt und langsam in 2 1 Eiswasser eingerührt. Der ausfallende schmierige Festkörper wurde abgetrennt, mit 400 ml Natriumhydrogencarbonat-Lösung und 150 ml Wasser gewaschen und im Vakuum getrocknet. Auswaage 34,3g (56,6%), Schmp. 92-93°C, Reinheit 98% (GC), außerdem sind 1,4% Ausgangsmaterial und 0,6% Sulfoxid enthalten.

Die Wasserphase wurde mit Methyl-tert.-butylether extrahiert, über Natriumsulfat getrocknet und im Vakuum eingeengt.
Ausbeute 23,9 g (39,4%), Schmp. 89-90°C, Reinheit 94,9% (GC), außerdem sind 2,0% Ausgangsmaterial und 3,1% Sulfoxid enthalten.

Unter Berücksichtigung der Reinheit beider Fraktionen entspricht dies einer isolierten Ausbeute von 93%.

### Stufe b:

### Herstellung von 2-Chlor-3-(4,5-dihydroisoxazol-3-yl)-4-methylthiobenzoesäure

b) Unter Stickstoff wurden zu 9,3 g (30,3 mmol) 3-(3-Brom-2-chlor-6-methylthiophenyl)-4,5-dihydroisoxazol gelöst in 75 ml Tetrahydrofuran bei Raumtemperatur 19,0 ml (38,0 mmol) einer 2-molaren Lösung von Isopropylmagnesiumchlorid in Tetrahydrofuran getropft. Dabei erwärmte sich die Mischung auf 40°C. Es wurde 2 h bei Raumtemperatur nachgerührt, portionsweise mit 20 g Trockeneis versetzt, wobei die Temperatur auf 10°C fiel und mit 10%iger Salzsäure auf einen pH-Wert von 0-1 angesäuert, wobei die Temperatur durch externe Kühlung unter 20°C gehalten wurde. Anschließend wurden 20 ml konzentrierte Kochsalzlösung zugegeben und mit Ethylacetat extrahiert, über Natriumsulfat getrocknet und im Vakuum eingeengt. Ausbeute 8,2 g Feststoff.
   Reinheit ca. 80% (¹H-NMR). Zur Reinigung wurde eine Probe von 0,66 g in 15 ml 15%iger Natronlauge gelöst, mit 10 ml Methylenchlorid gewaschen, mit conc. Salzsäure auf einen pH-Wert von 0-1 angesäuert, das Produkt abfiltriert und im Vakuum getrocknet. Ausbeute 0,45 g (68%) weißer Festkörper vom Schmp. 198-200°C. Reinheit >95% (¹H-NMR). Umgerechnet auf den ganzen Ansatz entspricht dies einer isolierten Ausbeute von ca. 68%. ¹H-NMR (DMSO-d₆, ppm): δ = 2,53 (s); 3,25 (t); 4,48 (t); 7,43 (d); 7,87 (d); 13,4 (verbr.).
bb) 8,3 kg (342 mol) Magnesium wurden 1,5 h bei 60 °C im Vakuum (100 mbar) getrocknet. Es wurde 14 1 Tetrahydrofuran zugefahren und bei 60 °C die Reaktion mit 500 ml 1,2-Dibromethan gestartet. Der Kesselinhalt fing an zu Sieden. Anschließend wurden 24,3 kg (310 mol) Isopropylchlorid in 128 l Tetrahydrofuran innerhalb 2 Stunden unter Siedekühlung zugefahren. Nach Zulaufende wurde der Ansatz 8 Stunden unter Rückfluß gekocht. Anschließend wurde unter Stickstoff auf 10 °C abgekühlt. Eine Lösung von 79,2 kg (258 mol) 3-(3-Brom-2-chlor-6-methylthiophenyl)-4,5-dihydroisoxazol gelöst in 215 1 Tetrahydrofuran wurde unter Kühlung innerhalb von 2 Stunden bei 10-26°C zudosiert. Es wurde 35 Minuten bei 20-25 °C nachgerührt. Anschließend wurden 23 kg Kohlendioxid (523 mol) innerhalb von 3.5 Stunden bei -7 bis 0 °C eingegast und über Nacht bei Eigentemperatur weitergerührt. Das Reaktionsgemisch wurde zur Hydrolyse innerhalb von 30 Minuten bei 10-26 °C zu einer Lösung von 42 1 31%iger Salzsäure in 172 1 Wasser zugefahren. Danach wurden 225 1 Tetrahydrofuran bei Normaldruck bis zu einer Innentemperatur von 67 °C abdestilliert. Anschließend wurde auf 60 °C abgekühlt und 380 1 Wasser zugefahren wobei das Produkt ausfiel. Restliches Tetrahydrofuran (160 1) wurde bei 500 mbar bis zu einer Innentemperacur von 78 °C abdestilliert. Das Reaktionsgemisch wurde auf 20 °C abgekühlt und abfiltriert. Das Produkt wurde mit 100 1 Wasser gewaschen und anschließend bei 50 °C mit 3 bar Stickstoff getrocknet. Ausbeute: 61 kg (87%) blaßbrauner Feststoff. Reinheit: >96% (HPLC).
bbb) Versuch mit Magnesiumpulver und Isopropylmagnesiumchlorid 235 mg (9,79 mmol) Magnesiumpulver (270 mesh) wurden unter Stickstoff im ausgeheizten Glaskolben mit 0,6 ml (1,2 mmol) einer 2-molaren Lösung von Isopropylmagnesiumchlorid in Tetrahydrofuran sowie mit 0,6 ml Tetrahydrofuran versetzt. Dabei stieg die Temperatur auf 33°C. Nun wurden 1 ml Tetrahydrofuran und 1 ml einer Lösung von 2,5 g (8,16 mmol) 3-(3-Brom-2-chlor-6-methylthiophenyl)-4,5-dihydroisoxazol in 12 ml Tetrahydrofuran (Lösung A) zugegeben. Dabei stieg die Reaktionstemperatur kurz auf 37°C. Die Mischung wurde auf 39°C erwärmt und weitere 4 ml der Lösung A zugetropft. Nach 15 min Rühren war die Temperatur auf 31°C gefallen und es wurden die restliche Lösung A und 3 ml Tetrahydrofuran hinzugefügt. Nach 15 min wurde auf 42°C erwärmt, bei dieser Temperatur 30 min gerührt, mit ca. 4 g Trockeneis versetzt, wobei die Temperatur auf -10°C fiel, 10 min nachgerührt und mit 10%iger Salzsäure auf einen pH-Wert von 0-1 angesäuert, wobei die Temperatur durch externe Kühlung unter 40°C gehalten wurde. Anschließend wurde 10 ml konzentrierte Kochsalzlösung zugegeben, mit Ethylacetat extrahiert, die vereinigten Extrakte mit Wasser gewaschen, über Natriumsulfat getrocknet und im Vakuum eingeengt. Ausbeute 2,21 g Feststoff. Reinheit ca. 75-80% (¹H-NMR). Ausbeute (Auswaage x Reinheit) ca. 75-80%.

### Stufe c:

### Herstellung von 2-Chlor-3-(4,5-dihydroisoxazol-3-yl)-4-methylsulfonylbenzoesäure

c) Zu 167 kg 2-Chlor-3-(4,5-dihydroisoxazol-3-yl)-4-methylthiobenzoesäure (616 mol) und 5,0 kg Natriumwolframat-Hydrat (15 mol) in 260 1 Eisessig wurden innerhalb von 5 Stunden bei 59 °C 200 1 30%igem Wasserstoffperoxid (1854 mol) zugefahren. Das Reaktionsgemisch erwärmte sich dabei auf 66 °C und wurde duch externe Kühlung bei dieser Tempereatur gehalten. Nach dem Ende der Zulaufzeit wurde 4 Stunden bei 60-65 °C nachgerührt.Anschließend wurden 113 1 Wasser bei 65 °C zugefahren und langsam auf 10 °C abgekühlt (5 °C/h) und über Nacht bei 10-15 °C nachgerührt. Der Kesselinhalt wurde abfiltriert, mit Wasser gewaschen, mit Stickstoffüberdruck vorgetrocknet und im Trockenschrank bei 60 °C getrocknet (100 mbar). Ausbeute: 170 kg (90,9%) farbloser Feststoff vom Schmp. 145-146°C. Die Substanz ist nach ¹H-NMR rein und direkt für die Wirkstoffsynthese geeignet.
   ¹H-NMR (DMSO-d₆, ppm): δ = 1,92 (s, Essigsäure); 3,30 (s), 3,33 (t); 4,52 (t); 8,07 (m); 12,3 (br.).

### Beispiel 7

### Herstellung von 2-Chlor-3-(4,5-dihydroisoxazol-3-yl)-4-methylsulfonylbenzoesäure (Eintopfvariante: Stufe b und c)

Es wurde bei 20-25 °C innerhalb von 10 Minuten zu 50 ml einer 2.0 M Isopropylmagnesiumchloridlösung in Tetrahydrofuran eine Lösung von 27,6 g (0,09 mol) 3-(3-Brom-2-chlor-6-methylthiophenyl)-4,5-dihydroisoxazol in 75 ml Tetrahydrofuran zugetropft und 30 Minuten nachgerührt. Bei -5 bis 5 °C wurden innerhalb 30 Minuten 5 g (0,114 mol) Kohlendioxid eingegast. Anschließend wurden bei 20-26 °C innerhalb 80 Minuten 209,7 g einer 12%igen Natriumhypochloritlösung zugetropft und 3,5 Stunden nachgerührt. Nachdem innerhalb 10 Minuten ein Gemisch aus 12,5 ml konz. Salzsäure und 62,5 ml Wasser zugetropft worden war, entstanden zwei klare Phasen. Die wäßrige Phase wurde abgetrennt und mit 80 ml Tetrahydrofuran extrahiert. Die beiden organischen Phasen wurden vereinigt. Anschließend wurde bei Normaldruck bis zu einer Innentemperatur von 100 °C das restliche Tetrahydrofuran entfernt und durch Wasser ersetzt. Das Reaktionsgemisch wurde auf Raumtemperatur abgekühlt wobei das Produkt ausfiel. Zum Schluß wurde mit Wasser gewaschen und getrocknet. Ausbeute: 24,2 g (86%) weißer Feststoff. Reinheit: >97% (HPLC).

## Patentansprüche

1. Verfahren zur Herstellung von schwefelhaltigen 2-Chlor-3-(4,5-dihydroisoxazol-3-yl)-benzoesäuren der allgemeinen Formel I 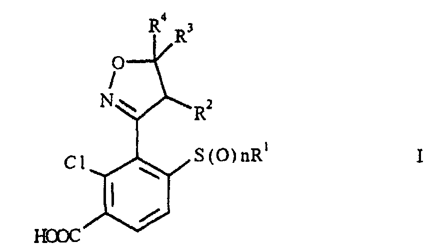 in der die Substituenten die folgende Bedeutung haben:
n 0, 1 oder 2;
R¹ C₁-C₆-Alkyl, C₁-C₆-Halogenalkyl;
R²,R³,R⁴ Wasserstoff, C₁-C₄-Alkyl, C₁-C₄-Halogenalkyl oder
R³ u. R⁴ bilden gemeinsam eine C₂-C₆-Alkandiyl-Kette, die einbis vierfach durch C₁-C₄-Alkyl substituiert sein kann,
**dadurch gekennzeichnet, daß** man
a) einen Thioether der Formel IV 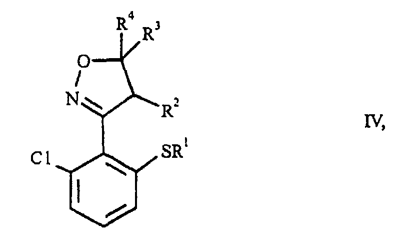 in der die Reste R¹ bis R⁴ die vorgenannte Bedeutung haben, mit einem Bromierungsmittel zum Bromthioether der Formel V 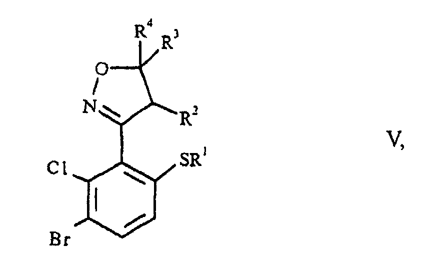 in der die Reste R¹ bis R⁴ die vorgenannte Bedeutung haben, bromiert und den Bromthioether V
b) mit Magnesiumpulver und/oder einer Grignardverbindung der Formel VI
R⁵Mg-Hal VI,
in der Hal für Cl, Br oder I und R⁵ für C₁-C₆-Alkyl stehen, in Gegenwart von Kohlendioxid zu Alkylthiobenzoesäuren der Formel Ia 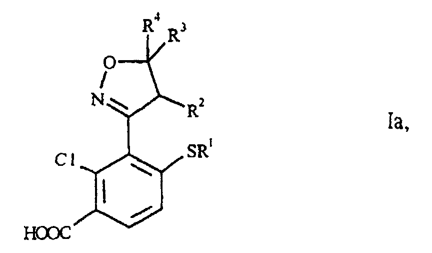 in der die Reste R¹ bis R⁴ die vorgenannte Bedeutung haben, umsetzt und zur Herstellung von Verbindungen I mit n 1 oder 2
c) mit einem Oxidationsmittel zu den entsprechenden Alkylsulfonyl- und Alkylsulfinylbenzoesäuren der Formel Ib 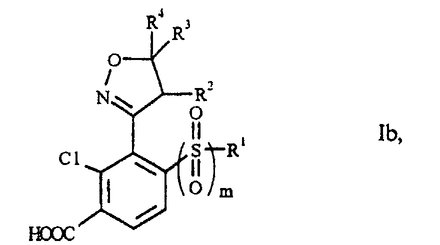 in der die Reste R¹ bis R⁴ die vorgenannte Bedeutung haben und m 0 oder 1 sein kann, oxidiert.

2. Verfahren zur Herstellung der Thioether der Formel IV gemäß Anspruch 1, **dadurch gekennzeichnet, daß** man 2,6-Dichlorbenzaldehyd mit einer Thiolverbindung der Formel III
R¹S-H III
in der R¹ die in Anspruch 1 gegebene Bedeutung hat, gegebenenfalls in Gegenwart einer Base zum Thioether der Formel VIII 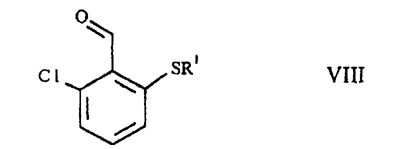 umsetzt und Thioether VIII dann mit Hydroxylamin ins Oxim IX 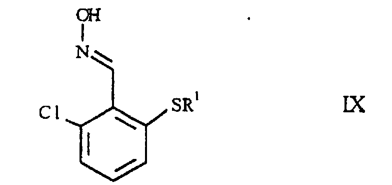 überführt, das schließlich mit einem Alken der Formel X 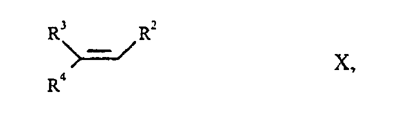 in der R² bis R⁴ die in Anspruch 1 genannte Bedeutung besitzen, in Gegenwart eines geeigneten Oxidationsmittels zur Reaktion gebracht wird.

3. Verfahren zur Herstellung von Thioethern der Formel IV gemäß Anspruch 1, **dadurch gekennzeichnet, daß** man das Oxim der Formel VII 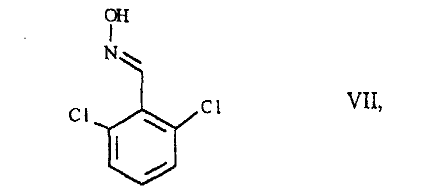 mit einer Thiolverbindung der Formel III gemäß Anspruch 2 zum Oxim IX umsetzt und Oxim IX schließlich mit einem Alken der Formel X gemäß Anspruch 2 in Gegenwart eines geeigneten Oxidationsmittels zur Reaktion bringt.

4. Verfahren zur Herstellung der Thioether der Formel IV gemäß Anspruch 1, **dadurch gekennzeichnet, daß** man das Oxim der Formel VII gemäß Anspruch 3 mit einem Alken der Formel X gemäß Anspruch 2 in Gegenwart eines geeigneten Oxidationsmittels zu 3-(2,6-Dichlorphenyl)isoxazolin der Formel II 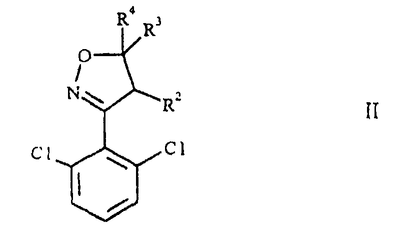 umsetzt und Verbindung II mit einer Thiolverbindung der Formel III gemäß Anspruch 2 in einem Lösungsmittel zur Reaktion bringt.

5. Verfahren gemäß Anspruch 1, **dadurch gekennzeichnet, daß** man in Stufe a) mit elementarem Brom in konzentrierter Schwefelsäure bei Temperaturen im Bereich von -10 bis 80°C umsetzt.

6. Verfahren gemäß Anspruch 1, **dadurch gekennzeichnet, daß** man in Stufe b) die Lösung eines Bromthioethers der Formel V in einem etherischen Lösungsmittel mit einer Suspension von 0 bis 2 Äquivalenten Magnesium und/oder 0,05 bis 1,2 Äquivalenten einer Grignardverbindung der Formel VI bei Temperaturen von 0 bis 50°C umsetzt und die intermediär gebildete Arylgrignardverbindung mit mindestens einem Äquivalent Kohlendioxid zur Reaktion bringt.

7. Verfahren gemäß Anspruch 1, **dadurch gekennzeichnet, daß** in Stufe c) Wasserstoffperoxid als Oxidationsmittel eingesetzt wird.

8. Alkylthiobenzoesäuren der allgemeinen Formel Ia 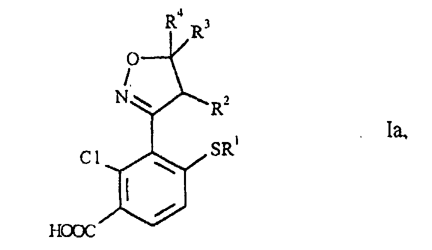 in der die Substituenten die folgende Bedeutung haben:
R¹ C₁-C₆-Alkyl, C₁-C₆-Halogenalkyl;
R²,R³,R⁴ Wasserstoff, C₁-C₄-Alkyl, C₁-C₄-Halogenalkyl oder
R³ u. R⁴ bilden gemeinsam eine C₂-C₆-Alkandiyl-Kette, die einbis vierfach durch C₁-C₄ Alkyl substituiert sein kann.

9. Bromthioether der Formel V 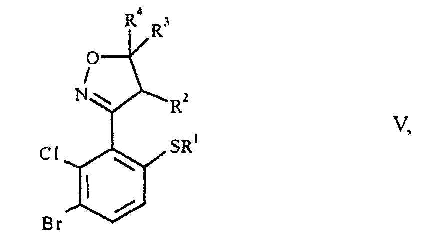 in der die Reste R¹ bis R⁴ die in Anspruch 1 genannte Bedeutung haben.

10. Thioether der Formel IV 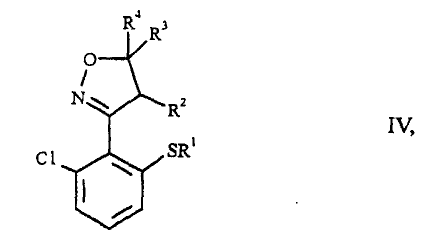 in der die Reste R¹ bis R⁴ die in Anspruch 1 genannte Bedeutung haben.

11. 3-(2,6-Dichlorphenyl)isoxazoline der Formel II 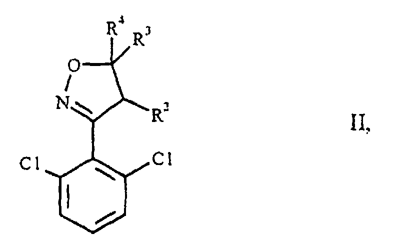 in der die Reste R² bis R⁴ die in Anspruch 1 genannte Bedeutung haben.

## Claims

1. A process for preparing sulfur-containing 2-chloro-3-(4,5-dihydro-3-isoxazolyl)benzoic acids of the formula I 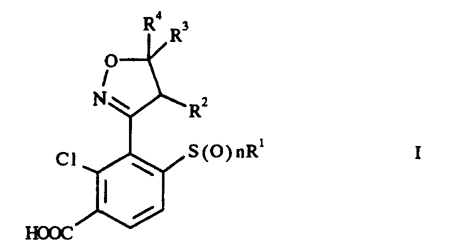 where the substituents have the following meanings:
n 0, 1 or 2;
R¹ C₁-C₆-alkyl, C₁-C₆-haloalkyl;
R²,R³,R⁴ hydrogen, C₁-C₄-alkyl, C₁-C₄-haloalkyl or
R³ and R⁴ together form a C₂-C₆-alkanediyl chain which may be substituted once to four times by C₁-C₄-alkyl,
which comprises
a) brominating a thioether of the formula IV 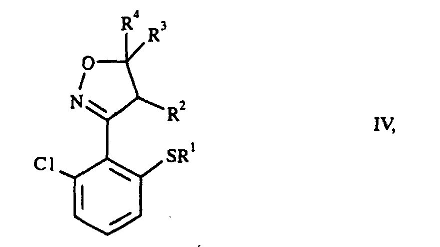 where R¹ to R⁴ have the abovementioned meanings,
with a brominating agent to give the bromo thioether of the formula V 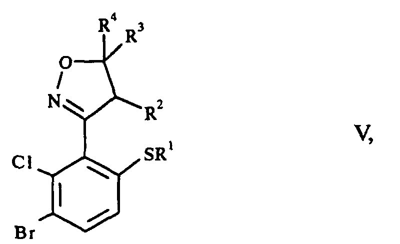 where R¹ to R⁴ have the abovementioned meanings, and reacting the bromo thioether V
b) with magnesium powder and/or a Grignard compound of the formula VI
R⁵Mg-Hal VI,
where Hal is Cl, Br or I and R⁵ is C₁-C₆-alkyl, in the presence of carbon dioxide to give alkylthiobenzoic acids of the formula Ia 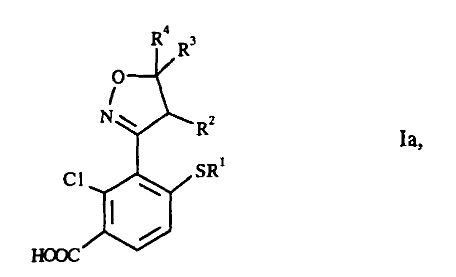 where R¹ to R⁴ have the abovementioned meanings, and to prepare compounds I with n 1 or 2
c) oxidizing with an oxidizing agent to the corresponding alkylsulfonyl- and alkylsulfinylbenzoic acids of the formula Ib 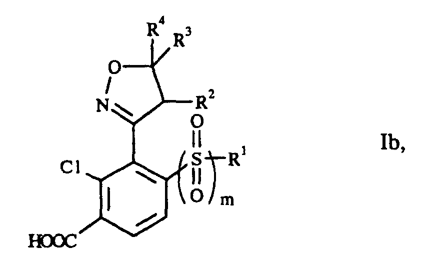 where R¹ to R⁴ have the abovementioned meanings, and m can be 0 or 1.

2. A process for preparing thioethers of the formula IV as set forth in claim 1, which comprises reacting 2,6-dichlorobenzaldehyde with a thiol of the formula III
R¹S-H III,
where R¹ has the meaning given in claim 1, in the presence or absence of a base, to give the thioether of the formula VIII 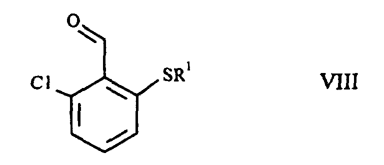 and then converting thioether VIII with hydroxylamine into the oxime IX 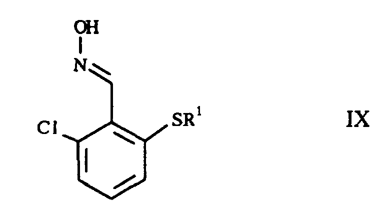 which is finally reacted with an alkene of the formula X 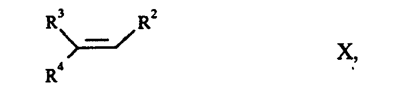 where R² to R⁴ have the meanings stated in claim 1, in the presence of a suitable oxidizing agent.

3. A process for preparing thioethers of the formula IV as set forth in claim 1, which comprises reacting the oxime of the formula VII 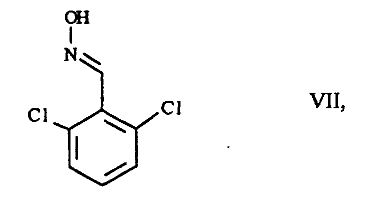 with a thiol of the formula III as set forth in claim 2 to give the oxime IX, and finally reacting oxime IX with an alkene of the formula X as set forth in claim 2 in the presence of a suitable oxidizing agent.

4. A process for preparing thioethers of the formula IV as set forth in claim 1, which comprises reacting the oxime of the formula VII as set forth in claim 3 with an alkene of the formula X as set forth in claim 2 in the presence of a suitable oxidizing agent to give 3-(2,6-dichlorophenyl)-isoxazoline of the formula II 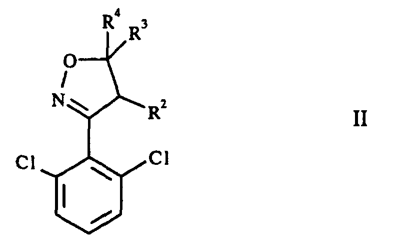 and reacting compound II with a thiol of the formula III as set forth in claim 2 in a solvent.

5. A process as claimed in claim 1, wherein the reaction in stage a) is carried out with elemental bromine in concentrated sulfuric acid at from -10 to 80°C.

6. A process as claimed in claim 1, wherein in stage b) the solution of a bromo thioether of the formula V is reacted in an ethereal solvent with a suspension of from 0 to 2 equivalents of magnesium and/or from 0.05 to 1.2 equivalents of a Grignard compound of the formula VI at from 0 to 50°C, and the intermediate aryl Grignard compound is reacted with at least one equivalent of carbon dioxide.

7. A process as claimed in claim 1, wherein hydrogen peroxide is employed as oxidizing agent in stage c).

8. An alkylthiobenzoic acid of the formula Ia 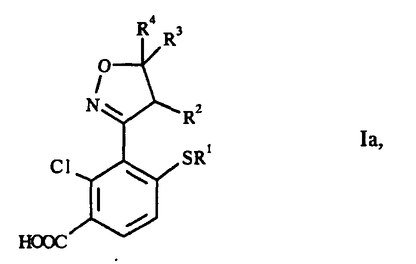 where the substituents have the following meanings:
R¹ C₁-C₆-alkyl, C₁-C₆-haloalkyl;
R²,R³,R⁴ hydrogen, C₁-C₄-alkyl, C₁-C₄-haloalkyl or
R³ and R⁴ together form a C₂-C₆-alkanediyl chain which may be substituted once to four times by C₁-C₄-alkyl.

9. A bromo thioether of the formula V 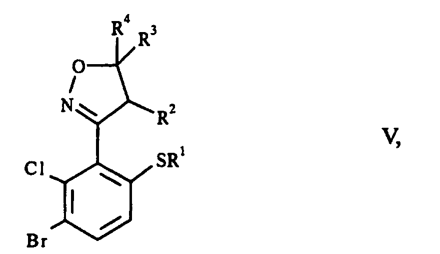 where R¹ to R⁴ have the meanings as specified in claim 1.

10. A thioether of the formula IV 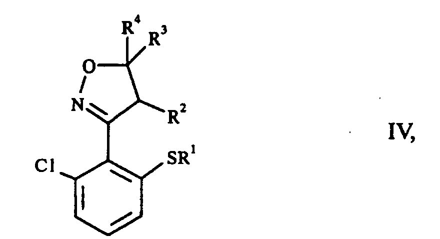 where R¹ to R⁴ have the meanings specified in claim 1.

11. A 3-(2,6-dichlorophenyl)isoxazoline of the formula II 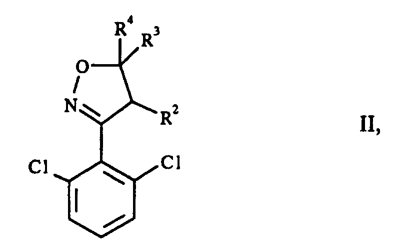 where R² to R⁴ have the meanings specified in claim 1.

## Revendications

1. Procédé pour la préparation d'acides 2-chloro-3-(4,5-dihydroisoxasole-3-yl)-benzoïques contenant du soufre, de formule générale I 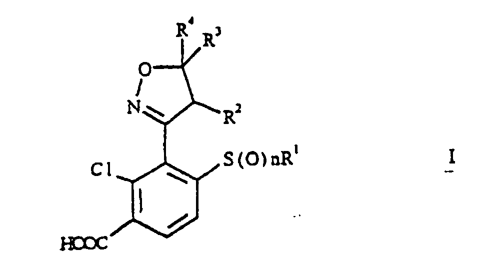 où les substituants ont la signification suivante:
n 0, 1 ou 2;
R¹ alkyle en C₁-C₆, halogénoalkyle en C₁-C₆;
R², R³, R⁴ hydrogène, alkyle en C₁-C₄, halogénoalkyle en C₁-C₄ ou
R³ et R⁴ forment ensemble une chaîne alcanediyle en C₂-C₆, qui peut être substituée une à quatre fois par des groupe alkyle en C₁-C₄,
**caractérisé par le fait qu'**on
a) brome un thioéther de formule IV 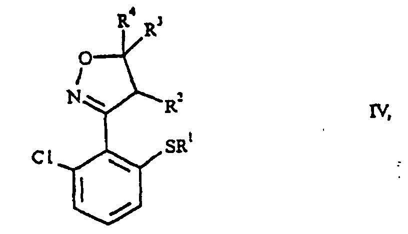 où les restes R¹ à R⁴ ont la signification susdite, avec un agent de bromation pour donner le bromothioéther de formule V 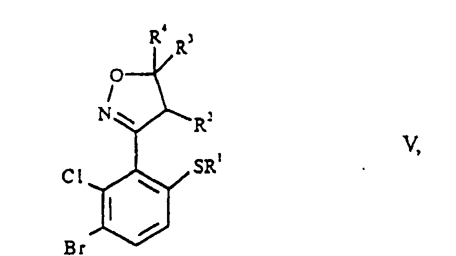 où les restes R¹ à R⁴ ont la signification susdite, et on fait réagir le bromothioéther V
b) avec de la poudre de magnésium et/ou un composé de Grignard de formule VI
R⁵Mg-Hal VI,
où Hal désigne Cl, Br ou I, et R⁵ représente un alkyle en C₁-C₆, en présence de dioxyde de carbone pour former des acides alkylthiobenzoïques de formule Ia 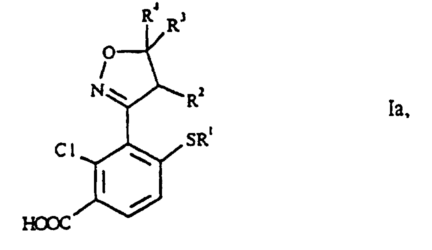 où les restes R¹ à R⁴ ont la signification susdite, et pour la préparation de composés I avec n valant 1 ou 2
c) on oxyde avec un agent d'oxydation pour former les acides alkylsulfonyl- et alkylsulfinylbenzoïques correspondants de formule Ib 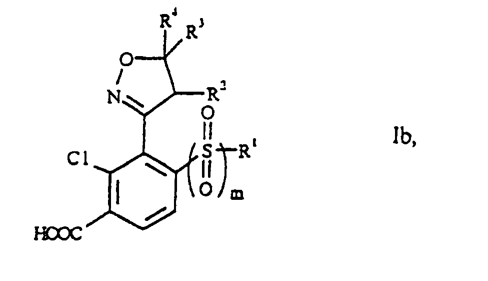
dans laquelle les restes R¹ à R⁴ ont la signification susdite et où m peut-être 0 ou 1.

2. Procédé pour la préparation des thioéthers de formule IV selon la revendication 1, **caractérisé par le fait qu'**on fait réagir du 2,6-dichlorobenzaldéhyde avec un composé thiol de formule III
R¹S-H III,
où R¹ a la signification indiquée dans la revendication 1, éventuellement en présence d'une base pour donner le thioéther de formule VIII 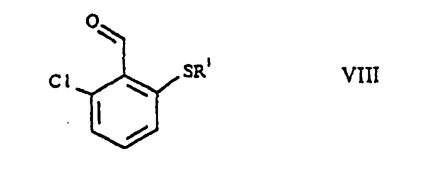 et on convertit le thioéther VIII avec de l'hydroxylamine en oxyme IX 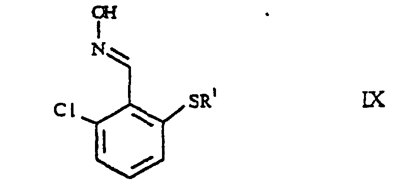 que l'on fait enfin réagir avec un akène de formule X 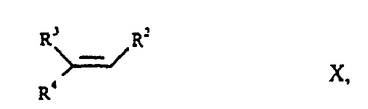 où R² à R⁴ possèdent la signification indiquée dans la revendication 1, en présence d'un agent d'oxydation approprié.

3. Procédé pour la préparation de thioéthers de formule IV selon la revendication I, **caractérisé par le fait qu'**on fait réagir l'oxime de formule VII 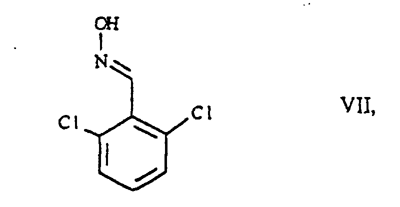 avec un composé thiol de formule III selon la revendication 2, pour obtenir l'oxime IX et on fait enfin réagir l'oxime IX avec un alcène de formule X selon la revendication 2 en présence d'un agent d'oxydation approprié.

4. Procédé pour la préparation des thioéthers de formule IV selon la revendication 1, **caractérisé par le fait qu'**on fait réagir l'oxime de formule VII selon la revendication 3 avec un alcène de formule X selon la revendication 2 en présence d'un agent d'oxydation approprié pour donner la 3-(2,6-dichlorophényl)isoxazoline de formule II 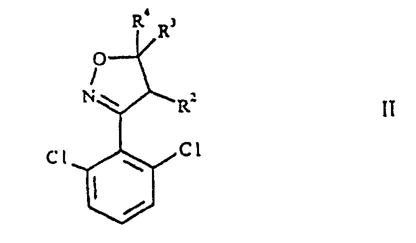 et on fait réagir le composé II avec un composé thiol de formule III selon la revendication 2 dans une solvant.

5. Procédé selon la revendication 1, **caractérisé par le fait qu'**on fait réagir dans l'étape a) avec du brome élémentaire dans de l'acide sulfurique concentré à des températures dans l'intervalle de --0 à 80°C.

6. Procédé selon la revendication 1, **caractérisé par le fait qu'**on fait réagir dans l'étape b) la solution d'un bromothioéther de formule V dans un solvant éthéré avec une suspension de 0 à 2 équivalents de magnésium et/ou 0,05 à 1,2 équivalents d'un composé de Grignard de formule VI à des températures de 0 à 50°C, et on fait réagir le composé d'aryl-Grignard formé à titre intermédiaire avec au moins un équivalent de dioxyde de carbone.

7. Procédé selon la revendication 1, **caractérisé par le fait que** dans l'étape c) on utilise du peroxyde d'hydrogène comme agent d'oxydation.

8. Acides alkylthiobenzoïques de formule générale Ia 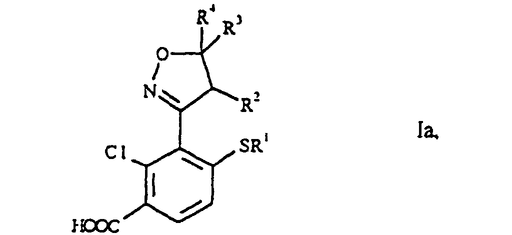 où les substituants ont la signification suivante:
R¹ alkyle en C₁-C₆, halogénoalkyle en C₁-C₆;
R², R³, R⁴ hydrogène, alkyle en C₁-C₄, halogénoalkyle en C₁-C₄ ou
R³ et R⁴ forment ensemble une chaîne alcanediyle en C₂-C₆, qui peut-être substituée une à quatre fois par des groupes alkyle en C₁-C₄.

9. Bromothioéthers de formule V 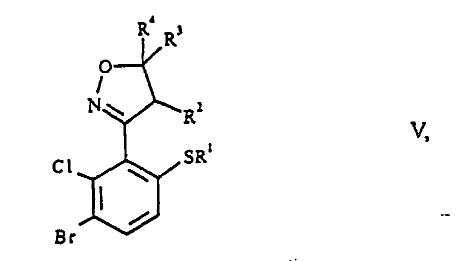 où les restes R¹ à R⁴ ont la signification indiquée dans la revendication 1.

10. Thioéthers de formule IV 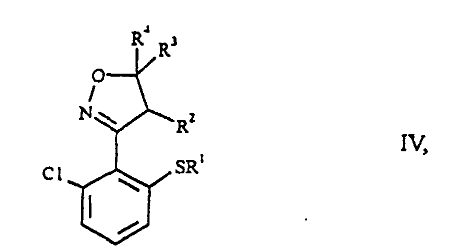 où les restes R¹ à R⁴ ont la signification indiquée dans la revendication 1.

11. 3-(2,6-dichlorophényl)isoxazoline de formule II 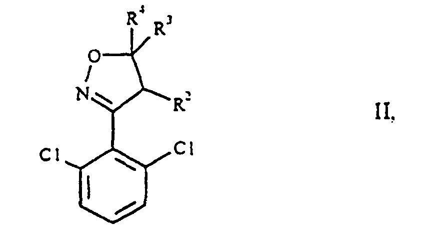 où les restes R² à R⁴ ont la signification indiquée dans la revendication 1.
